# EUROPÄISCHE PATENTSCHRIFT

(11) **EP 0 637 957 B1**
(45) Veröffentlichungstag und Bekanntmachung des Hinweises auf die Patenterteilung: **23.07.1997**
(21) Anmeldenummer: 93909844.8
(22) Anmeldetag: 27.04.1993
(51) Int. Cl.: A61K 31/20, A61K 31/23

(54) **VERWENDUNG EINER OMEGA-3-FETTSÄUREN ENTHALTENDEN EMULSION ZUR HERSTELLUNG EINES PARENTERAL ZU VERABREICHENDEN ARZNEIMITTELS ZUR BEHANDLUNG VON ENTZÜNDLICHEN ERKRANKUNGEN**
USE OF AN EMULSION CONTAINING OMEGA-3 FATTY ACIDS TO PRODUCE A MEDICAMENT TO BE PARENTERALLY ADMINISTERED FOR TREATING INFLAMMATORY DISEASES
UTILISATION D'UNE EMULSION CONTENANT DES ACIDES GRAS OMEGA-3 POUR LA FABRICATION D'UN MEDICAMENT ADMINISTRABLE PAR VOIE PARENTERALE, POUR LE TRAITEMENT DE MALADIES INFLAMMATOIRES

(30) Priorität: 28.04.1992 US 875243
(43) Veröffentlichungstag der Anmeldung: 15.02.1995
(73) Patentinhaber: Fresenius AG, 61350 Bad Homburg v.d.H (DE)
(72) Erfinder: ASKANAZI, Jeffrey 1230 Park Ave., Greenville MI 488389 (US); KATZ, David, P. 1230 Park Ave., New York, NY 10128 (US); SCHLOTZER, Ewald Fresenius AG, D-6370 Oberursel 1 (DE); FÜRST, Peter, D-7000 Stuttgart 1 (DE)
(74) Vertreter: Fuchs, Luderschmidt & Partner Patentanwälte
(86) Internationale Anmeldenummer: EP9301011
(87) Internationale Veröffentlichungsnummer: WO9321912

(56) Entgegenhaltungen:
- EP-A- 0 311 091
- WO-A-87/02247
- ANN. INTERN MED., Bd. 116, Nr. 8, 15. April 1992, Seiten 609-614; W.F. STENSON ET AL.: 'DIETARY SUPPLEMENTATION WITH FISH OIL IN ULCERATIVE COLITIS'
- ALIMENTARY PHARMACOLOGY & PHARMACEUTICS, Bd. 3, Nr. 5, 1989, Seiten 415-424; T. B. MCCALL: 'THERAPEUTIC POTENTIAL OF FISH OIL IN THE TREATMENT OF ULCERATIVE COLITIS'
- KIDNEY INTERNATIONAL, Bd. 36, Nr. 4, 1989, Seiten 653-660; W.F. CLARK ET AL.: 'OMEGA-3 FATTY ACID DIETARY SUPPLEMENTATION IN SYSTEMIC LUPUS ERYTHEMATOSUS'
- ANN. RHEUM. DIS., Bd. 50, Nr. 7, 1991, Seiten 463-466; A.J.E. WALTON ET AL.: 'DIETARY FISH OIL AND THE SEVERITY OF SYMPTOMS IN PATIENTS WITH SYSTEMIC LUPUS ERYTHEMATOSUS'
- J. NUTR., Bd. 119, Nr. 4, 1989, Seiten 521-528; A.P. SIMOPOULOS: 'SUMMARY OF THE NATO ADVANCED RESEARCH WORKSHOP ON DIETARY OMEGA3 AND OMEGA6 FATTY ACIDS: BIOLOGICAL EFFECTS AND NUTRITIONAL ESSENTIALITY'

## Beschreibung

Gegenstand der vorliegenden Erfindung ist die Verwendung einer Emulsion, die eine oder mehrere mehrfach ungesättigte, langkettige Omega-3-Fettsäuren oder deren pharmazeutisch verträgliche Ester oder Salze sowie übliche Hilfs- und Zusatzstoffe enthält, zur Herstellung eines parenteral zu verabreichenden Arzneimittels zur Behandlung von Colitis ulcerosa.

Entzündliche Erkrankungen sind pathologische Prozesse, die aus dynamischen Komplexen von zytologischen und histologischen Reaktionen bestehen, die in den betroffenen Blutgefäßen und benachbarten Geweben in Reaktion auf Verletzung oder durch physikalische, chemische oder biologische Mittel verursachte abnormale Stimulation ablaufen (vgl. Stedman's Medical Dictionary, S. 781, 25. Ausgabe 1990, Definition der Entzündung). Es existieren viele Arten von entzündlichen Erkrankungen, wie entzündliche Lungenerkrankungen, entzündliche Darmerkrankungen oder systemischer Lupus erythematodes.

Colitis ulcerosa stellt eine entzündliche Darmerkrankung dar.

Entzündliche Darmerkrankungen oder Funktionsstörungen sind jene physiologischen Zustände, bei denen eine Entzündung der Magendarmschleimhaut vorliegt. Entzündliche Darmerkrankungen werden gewöhnlich von Abdominalschmerz, Diarrhoe und nachfolgender Dehydratation und Auftreten von Rektalblutung begleitet. Gastrointestinale Infektionen können stattfinden und sich wiederholen. Patienten mit entzündlicher Darmerkrankung leiden häufig unter mangelhafter Absorption von Nährstoffen und Unterernährung, welche die Darmfunktion verschlimmern und verschlechtern. Mangelhafte Absorption erfolgt aufgrund einer Herabsetzung der Fähigkeit der Dünndarmmukosa, multiple Nährstoffe zu absorbieren. Die Unfähigkeit der Dünndarmmukosa, Nährstoffe zu absorbieren, kombiniert mit dem Verlust von Flüssigkeit, Elektrolyten, Protein und Blut in diarrhoischen Stühlen kann einen Patienten mit entzündlicher Darmerkrankung stark entkräften.

Bei Patienten mit entzündlichen Erkrankungen, die unterernährt sind oder nicht in der Lage sind, Energie in ausreichender Menge oral aufzunehmen, ist es Standardpraxis, diesen Patienten Energie in Form von intravenösen Fettemulsionen durch parenterale Verabreichung, die dem Patienten große Mengen an Omega-6-Fettsäuren liefern, zuzuführen. Die parenterale Verabreichung von Omega-6-Fettsäuren enthaltenden Fettemulsionen an diese Patienten verhindert oder verbessert den Mangel an essentiellen Fettsäuren. Diese Emulsionen sind sichere und bevorzugte Energiequellen.

Durch parenterale Verabreichung von Omega-6-Fettsäuren enthaltenden Fettemulsionen an einige Patienten mit Mukoviszidose, wurde z.B. eine Verbesserung im Energiegefühl der Patienten und des Wohlbefindens, der Belastungstoleranz, der Sauerstoffaufnahme (VO₂) und eine Verringerung der Sekretionen mit einer sichtbaren Reduktion des pulmonalen Entzündungsprozesses festgestellt (Nutrition International, 3, 277-279 (1987), Crit. Care Med., 15, 960-964 (1987)). Im Bezug auf Patienten mit Mukoviszidose hat die klinische Erfahrung gezeigt, daß während eine Anzahl von Patienten, denen parenteral Omega-6-Fettsäuren enthaltende Fettemulsion verabreicht wurde, positiv beeinflußt wurde und Verbesserung zeigte, eine signifikante Anzahl von Patienten überraschend nicht reagierte.

Parenteral zu verabreichende, Omega-6-Fettsäuren enthaltende Standard-Fettemulsionen, die den Patienten gegeben werden, sind von pflanzlichen Ölen, wie Safloröl, Sojabohnenöl oder Gemischen derselben abgeleitet und sind kommerziell unter den Handelsnamen "Intralipid", "Lipovenös" oder "Liposyn" verfügbar. Diese Fettemulsionen enthalten mindestens 40-50 % Linolsäure (C_{18:2}), eine Omega-6-Fettsäure, die als essentielle Fettsäure bekannt ist und als Hauptbestandteil von Säugetierzellmembranen für das Wachstum, die Entwicklung und die Aufrechterhaltung der normalen Zellfunktion notwendig ist. Linolsäure (C_{18:2}) ist außerdem ein Präcursor für eine andere Omega-6-Fettsäure, die Arachidonsäure (C_{20:4}), welche als Präcursor für die Synthese von Prostaglandinen, Leukotrienen und anderen Hydroperoxyarachidonat-Derivaten dient.

Prostaglandine und Leukotriene sind an Entzündungsreaktionen, Plättchenaggregation und anderen Immunreaktionen beteiligt. Von Arachidonsäure abgeleitete Prostaglandine verursachen Plättchenaggregation. Leukotriene können viele der pathophysiologischen Merkmale von Asthma, chronischer Bronchitis und Mukoviszidose produzieren. Z. B. fördert Leukotrien B₄ die Aggregation und Adhäsion von Neutrophilen an vaskuläres Endothel, eine frühe Stufe in der Entwicklung einer Entzündung. Leukotrien C₄ und D₄ sind die Hauptkomponenten der langsam reagierenden Substanz der Anaphylaxie (SRS-A) und scheinen bei Bronchialasthma, chronischer Bronchitis und Mukoviszidose involviert zu sein.

Nicht-reagierende Patienten waren jene Patienten mit Mukoviszidose, die nicht auf die parenterale Verabreichung von Omega-6-Fettsäuren enthaltenden Fettemulsionen reagierten. Bei nicht reagierenden Patienten wurde beobachtet, daß Linolsäure im Plasma anstieg, daß jedoch keine Erhöhung der anderen Omega-6-Fettsäuren, die kettenelongiert und desaturiert sind, besonders Dihomogammalinolensäure (20:3, Omega-6, DHLA) stattfand, was eine metabolische Blockierung des Enzyms, das für diese Umwandlung verantwortlich ist, anzeigt.

Omega-3-Fettsäuren, die in Fischölen von Kaltwasser-Meerestieren (z.B. Dorsch, Hering, Makrelen, Menhaden, Lachs, Sardinen, Anschovis) gefunden werden, sind lineare, polyungesättigte Säuren mit 20 bis 24 Kohlenstoffatomen und 5 bis 7 Doppelbindungen. Omega-3-Fettsäuren umfassen Eicosapentaensäure (C_{20:5}) und Docosahexaensäure (C_{22:6}), welche Präcursoren für Trien-Prostaglandine und Pentaen-Leukotriene sind und eine von ihren Omega-6-Äquivalenten verschiedene Wirksamkeit besitzen. Omega-3-Fettsäuren sind in Zellmembranen und im Serum, ähnlich wie die Omega-6-Fettsäuren, inkorporiert.

Das in Zellmembranen und im Plasma eines Säugetieres gefundene Verhältnis von Omega-3-Fettsäuren zu Omega-6-Fettsäuren hängt davon ab, ob die Fettsäuren durch Aufnahme von Nahrungsfett, wie Fleischfetten oder pflanzlichem Öl, die einen hohen Gehalt an Omega-6-Fettsäuren und einen niedrigen Gehalt an Omega-3-Fettsäuren aufweisen, oder aus Fischöl, welches eine große Menge an Omega-3-Fettsäuren aufweist, erhalten werden. Die Arten von Prostaglandinen und Leukotrienen, die gebildet werden, stehen entsprechend mit der Menge der Omega-6-Fettsäuren und Omega-3-Fettsäuren, die als Präcursorsubstrate verfügbar sind, in Beziehung. Dies ist auf die Tatsache zurückzuführen, daß die Cyclooxygenase, das erste Enzym im Prostaglandin-Syntheseweg, und die Lipoxygenase, das erste Enzym im Leukotrien-Syntheseweg, sowohl Arachidonsäure (C_{20:4}) als auch Eicosapentaensäure (C_{20:5}) als Substrat verwenden können. Falls mehr Omega-3-Fettsäuren verfügbar sind, werden sie die Enzyme, welche normalerweise Omega-6-Fettsäuren verwenden, kompetitiv inhibieren, was zu herabgesetzten Spiegeln von Prostaglandinen und Leukotrienen, die aus Omega-6-Fettsäuren gebildet werden, führt. In Neutrophilen z.B. wird Arachidonsäure (C_{20:4}) gewöhnlich mittels Lipoxygenase zu Leukotrien B₄ metabolisiert, das die Aggregation und Adhäsion der Neutrophilen an das vaskuläre Endothel, den frühen Stadien der Entzündung, fördert. Eicosapentensäure (C_{20:5}) ist jedoch ein bevorzugtes Substrat für die Lipoxygenase-Wege in Neutrophilen und verdrängt die Arachidonsäure (C_{20:4}). Eine solche Verdrängung führt zu geringeren Mengen Leukotrien B₄ und reduzierter Chemotaxie und Adhäsion an die endothelialen Zellen, folglich zu einer Reduzierung der Entzündung.

Hinsichtlich der Effekte einer durch Fischöl supplementierten Diät bezüglich der Verhinderung und Behandlung cardiovaskulärer, entzündlicher und immunologischer Störungen wurden Studien durchgeführt. Patienten mit cardiovaskulärer Erkrankung, die eine an Fisch und Fischöl reiche Diät einnahmen, zeigten eine Abnahme der Spiegel an Cholesterin und Triglyceriden. Mäuse mit Lupus-artiger autoimmuner Nephritis entwickeln eine Proteinurie und sterben frühzeitig. Würden jedoch solche Mäuse mit einer mit Menhaden-Fischöl supplementierten enteralen Diät gefüttert, so wurden Proteinurie und frühzeitiger Tod verzögert, was zu einem verlängerten Überleben führte (V. Kelley et al., J. Immunol., 134(3):1914-1919 (1985); J.D. Prickett et al., J. Clin. Invest., 68:556-559 (1981); D.R. Robinson et al., Arthritis and Rheumatism, 29:539-546 (1986). Ratten mit induzierter granulomatöser Colitis, die mit einer enteralen Diät mit geringem Fettgehalt, die mit 8 Gew.-% Dorschleberöl, das Omega-3-Fettsäuren enthielt, supplementiert war, gefüttert wurden, zeigten eine Reduzierung der Stärke der entzündlichen Schädigungen des Colons, verglichen mit Ratten, die mit einer enteralen Diät mit geringem Fettgehalt, die mit 8 Gew.-% Sonnenblumenöl, das Omega-6-Fettsäuren enthielt, supplementiert war, gefüttert wurden (J. Vilaseca et al., Gut, 31, 539-544 (1990)). Mit Fischöl supplementierte Diäten haben auch Patienten mit Psoriasis und atopischer Dermatitis positiv beeinflußt, zeigten jedoch unbestimmte Vorteile in der Behandlung von aktiver rheumatoider Arthritis.

Fettemulsionen und Nabrungssupplements, die Omega-3-Fettsäuren enthalten, wurden beschrieben und sind in der Technik bekannt.

Die US-PS 4,678,808 offenbart eine Fettemulsion für die intravenöse Therapie für die Behandlung einer thrombotischen Erkrankung, d.h. Bildung von Blutgerinnseln in Blutgefäßen oder im Herzen. Die Fettemulsion enthält einen Emulgator, Wasser und ein Öl von Meerestieren, das einen Omega-3-Fettsäureester in einer Konzentration zwischen 5 und 50 % (Gew./Volumen), vorzugsweise 10 bis 20 % enthält, wobei die Konzentration der freien Fettsäure in der Emulsion unter 5 mäq/l beträgt. In der US-PS wird erwähnt, daß Fettemulsionen, die Öl von Meerestieren enthalten, für die Behandlung von Störungen vorgeschlagen wurden, die mit Imbalancen von Arachidonsäuremetaboliten, einschließlich autoimmunen Syndromen, akuten und chronischen entzündlichen Erkrankungen einhergehen, wie Psoriasis und akutes Atemnotsyndrom (ARDS), Atherosclerose, Schlaganfall, Myocardinfarkt, Tiefvenenthrombose und andere cardiovaskuläre Erkrankungen (Spalte 4, Zeile 8-15), lehrt jedoch nicht, in welcher Weise entzündliche Erkrankungen mit Omega-3-Fettsäure enthaltenden Fettemulsionen behandelt werden sollen.

Die US-PS 4,820,731 offenbart ein Nahrungssupplement, das einen Emulgator, Wasser und eine Ölfraktion, die 10 bis 20 Gew.-% des Supplementes ausmacht und aus einem Gemisch von Ölen, die reich an Omega-3-Fettsäuren (10 bis 90 Gew.-% der Ölfraktion) und Ölen, die reich an Omega-6-Fettsäuren sind, besteht, enthält. Die US-PS offenbart ebenfalls eine Methode zum Minimieren der Effekte von Infektion und Minimieren der Effekte von nachfolgender Infektion bei gefährdeten Lebewesen durch orales oder intravenöses Verabreichen dieses Nahrungssupplementes. Die US-PS enthält jedoch keine Offenbarung der Behandlung von entzündlichen Erkrankungen mit Omega-3-Fettsäure enthaltenden Fettemulsionen.

Die US-PS 5,089,268 offenbart eine sterile Fettemulsion für die parenterale Verwendung. Die Fettemulsion enthält von 1 bis 30 Gew.-% eines Neutralfettgemisches von Fettsäuretriglyceriden, die Omega-3-Fettsäuren, Omega-6-Fettsäuren, mittelkettige Triglyceride oder Gemische derselben enthalten, von 1 bis 4 Gew.-% eines Fischöl angereichterten Eigelbphosphatit-Emulgators, der aus Eiern von Hühnern erhalten wurde, deren Diät mit Fischölen, die reich an Eicosapentaensäure, Docosahexaensäure oder Gemischen derselben waren, supplementiert worden war, bis zu 2,25 Gew.-% eines Osmolalitäts-Modifikators und steriles Wasser. In der US-PS wird kurz erwähnt, daß von Omega-3-Fettsäuren bekannt ist, daß sie gewissen therapeutischen Wert bei der Behandlung von Herzkrankheit, entzündlichen Störungen und Infektion besitzen, es wird jedoch gelehrt, daß es nicht wünschenswert ist, gestreßten und/oder unterernährten Patienten große Mengen Omega-3- und Omega-6-Fettsäuren in der Form von Neutralfetten zu geben (vgl. Spalte 1, Zeilen 57-65). In der US-PS wird nicht die Behandlung von entzündlichen Erkrankungen mit Omega-3-Fettsäure enthaltenden Fettemulsionen offenbart.

Es wurde gezeigt, daß Fischöl angereicherte Diäten nachteilige Effekte bei Patienten mit pulmonalen Erkrankungen produzieren. Meerschweinchen, die mit einem Nahrungssupplement von raffiniertem Menhaden-Fischöl gefüttert wurden, erlitten größere Bronchokonstriktion während induzierter Anaphylaxie und erlitten erhöhte Anaphylaxie als Meerschweinchen, die mit Rinderfett gefüttert wurden (T.H. Lee et al., Am. Rev. Respir. Dis., 132:1204-1209 (1985)). Aspirin- intolerante Patienten mit Asthma, die mit einer Fischöl angereicherten Diät ernährt wurden, zeigten eine Zunahme der Bronchialobstruktion, eine Verschlechterung im Spitzen-Expirationsstrom (peak expiratory flow) und eine Zunahme in der Verwendung von Bronchodilatoren (C. Picado et al., Thorax, 43:93-97 (1988)).

Die Aufgabe der vorliegenden Erfindung besteht daher darin, für die Behandlung von Colitis ulcerosa, insbesondere für Patienten, die nicht auf die parenterale Verabreichung von Omega-6-Fettsäuren enthaltenden Fettemulsionen reagieren, eine Alternative bereitzustellen, um die Vorteile und Verbesserungen ihrer Gesundheit zu erreichen, die bei reagierenden Patienten erzielt werden.

Gelöst wurde erfindungsgemäß diese Aufgabe durch die Verwendung einer Emulsion, die eine oder mehrere mehrfach ungesättigte, langkettige Omega-3-Fettsäuren oder deren pharmazeutisch verträgliche Ester oder Salze enthält, zur Behandlung von Colitis ulcerosa.

Erfindungsgemäß wird eine Möglichkeit zur Umgehung der metabolischen Blockierung bei den nicht auf Omega-6-Fettsäure enthaltende Emulsionen reagierenden Patienten und zur Erzielung der therapeutischen Vorteile für die nicht-reagierenden Patienten, wie sie bei reagierenden Patienten erzielt werden, bereitgestellt, insbesondere werden durch die erfindungsgemäße Verwendung den nicht-reagierenden Patienten Fettsäuren über einen völlig verschiedenen biochemischen Weg geliefert und wird die Synthese von Prostaglandinen und Leukotrienen, die von Omega-3-Fettsäure-Präcursoren abgeleitet sind, nicht jedoch von Omega-6-Fettsäure-Präcursoren, die im allgemeinen für diese Patientenpopulation nachteilig ist, ausgenützt.

Die erfindungsgemäß verwendete Fettemulsion kann Mensch und Tier, vorzugsweise dem Menschen parenteral, z.B. intravenös, zur Behandlung von Colitits ulcerosa, verabreicht werden.

Die erfindungsgemäß verwendete Emulsion enthält bevorzugt solche mehrfach ungesättigten langkettigen Omega-3-Fettsäuren, die 18 bis 22 Kohlenstoffatome enthalten, und/oder deren pharmazeutisch verträglichen Ester und Salze. Beispiele für geeignete Omega-3-Fettsäuren sind Eicosapentaensäure (EPA) und Docosahexaensäure (DHA), wobei bevorzugt Eicosapentaensäure verwendet wird. In den Emulsionen können eine oder mehrere der Omega-3-Fettsäuren vorhanden sein. Beispielsweise können Gemische aus Eicosapentaensäure (C_{20:5}) und Docosahexansäure (C_{22:6}) verwendet werden. Die pharmazeutisch verträglichen Ester und Salze der genannten Omega-3-Fettsäuren werden erfindungsgemäß bevorzugt verwendet, wobei die pharmazeutisch verträglichen Ester der Säuren besonders bevorzugt sind. Pharmazeutisch verträgliche Ester der Omega-3-Fettsäuren sind Ethylester und Glycerinester, z.B. Mono-, Di- oder Triglycerinester, wobei Triglyceride bevorzugt sind. Als pharmazeutisch verträgliche Salze der Säuren geeignet sind, z.B. die Natriumsalze.

In den erfindungsgemäß verwendeten Emulsionen können die Omega-3-Fettsäuren, deren pharmazeutisch verträglichen Ester oder Salze in Reinform oder als Bestandteil von Ölen, wie natürlichen, aufbereiteten, semisynthetischen oder synthetischen Ölen enthalten sein. Vorzugsweise werden Fischöle eingesetzt. Erfindungsgemäß werden unter "Fischölen" natürliche Fischöle, behandelte Fischöle oder hochgereinigte Fischölkonzentrate verstanden. So können erfindungsgemäß auch behandelte Fischöle verwendet werden, wie sie z.B. in der DE-PS 37 22 540, auf die aus Offenbarungsgründen in vollem Umfang Bezug genommen wird, beschrieben sind.

Die Omega-3-Fettsäuren, deren pharmazeutisch verträglichen Ester oder Salze sind in den erfindungsgemäß verwendeten Emulsionen in Mengen von 1 bis 40 Gew.-%, vorzugsweise 2 bis 35 Gew.-% und insbesondere 3 bis 30 Gew.-% enthalten.

Die erfindungsgemäß verwendeten Emulsionen enthalten ebenfalls mindestens einen physiologisch unbedenklichen Emulgator. Geeignet sind Phospholipide tierischen oder pflanzlichen Ursprungs, wie Eilecithin, Eiphosphatite, Sojaphosphatite, Sojalecithin und andere Phospholipide. Vorzugsweise werden solche Phospholipide eingesetzt, die als ungesättigte Fettsäure EPA enthalten. Insbesondere geeignet ist Eilecithin. Geeignet sind erfindungsgemäß auch Gemische von zwei oder mehreren Emulgatoren.

Der Emulgator liegt in der Emulsion in einer Menge von 0,1 bis 6 Gew.-%, vorzugsweise in einer Menge von 0,4 bis 2 Gew.-% vor.

Die Emulsionen können ferner Vitamin E, z.B. in Form von Tocopherol oder pharmazeutisch unbedenklichem Tocopherolester, z.B. Tocopherolacetat, in einer Menge von 0,15 bis 1,5 Gew.-% (bezogen auf den Fettgehalt) als Antioxidans enthalten.

Als weitere Zusätze können die erfindungsgemäß verwendeten Emulsionen übliche Hilfs- und Zusatzstoffe, wie übliche Emulsionsstabilisatoren, Isotonisierungszusätze und/oder Coemulgatoren aufweisen.

Als Isotonisierungszusätze geeignet sind die üblicherweise verwendeten Isotonisierungsmittel, wie z.B. Glycerin, Glucose, Xylit oder Sorbit, wobei Glycerin bevorzugt ist. Die Menge dieser Isotonisierungszusätze in der Emulsion beträgt geeigneterweise 1 bis 5 Gew.-%, vorzugsweise 2,5 Gew.-%.

Bevorzugt sind erfindungsgemäß solche Emulsionen, die Öle, vorzugsweise Fischöle, Glycerin, mindestens einen Emulgator und gereinigtes Wasser enthalten, wobei die Öle mindestens 20 Gew.-% Omega-3-Fettsäuren enthalten. Geeignet sind erfindungsgemäß Öle, vorzugsweise Fischöle,wie sie vorstehend definiert wurden, die - bezogen auf die Gesamtfettsäuren - 20 bis 100 Gew.-% Omega-3-Fettsäuren, vorzugsweise 30 bis 100 Gew.-% Omega-3-Fettsäuren und insbesondere 40 bis 100 Gew.-% Omega-3-Fettsäuren enthalten. Die Omega-3-Fettsäuren sind hauptsächlich Eicosapentaensäure (C_{20:5}), Docosahexaensäure (C_{22:6}) und Gemische derselben.

Unter "gereinigtem Wasser" wird hierin Wasser für Injektionszwecke verstanden.

Die Fischöle werden aus Kaltwasser-Meerestieren, wie Dorsch, Hering, Makrele, Menhaden, Lachs, Sardinen, Anschovis erhalten. Der Gehalt an Omega-3-Fettsäuren in den Fischölen variiert abhängig von der Quelle der Fischöle und kann durch Raffinierung der Fischöle entsprechend erhöht werden. Die Fischöle sind geeigneterweise hochraffiniert und enthalten mindestens 20 Gew.-% Omega-3-Fettsäuren, bezogen auf die Gesamtfettsäuren des Fischöls.

Die in der Emulsion verwendete Menge an Fischölen hängt von der Dosierung, dem Prozentsatz der Omega-3-Fettsäuren und der Gesamt-Fettkonzentration der Emulsion ab. Therapeutische Dosen richten sich nach dem Körpergewicht und der Infusionsdauer. Die Konzentration der Fischöle in der Emulsion variiert zwischen etwa 3 bis 30 Gew.-% der Emulsion, wobei 10 bis 20 Gew.-% für die Konzentration der Fischöle in der Emulsion bevorzugt ist.

Die pharmazeutisch wirksame Menge an Omega-3-Fettsäuren für die Behandlung von entzündlichen Erkrankungen liegt geeigneterweise im Bereich von 1 bis 30 g Omega-3-Fettsäuren pro Tag, vorzugsweise im Bereich von 1 bis 20 g Omega-3-Fettsäuren pro Tag und insbesondere im Bereich von 1 bis 15 g Omega-3-Fettsäuren pro Tag und kann beispielsweise 4 bis 5 g oder 1 bis 10 g oder 14 bis 15 g Omega-3-Fettsäuren pro Tag betragen.

Verwendbare Emulgatoren sind die vorstehend genannten Emulgatoren, wobei die Konzentration des Emulgators von der Ölmenge in der Emulsion abhängt. Die Konzentration kann im Bereich von etwa 0,1 bis 6 Gew.-% der Emulsion liegen. Die bevorzugten Konzentrationen der Emulgatoren betragen etwa von 0,4 bis 2,0 %, wenn die Ölmenge zwischen 10 und 20 % liegt.

Die Emulsionen werden in Glas- oder Plastikbehälter abgefüllt, dann sterilisiert und bis zur Verwendung gelagert.

In der Fettemulsion liegt die Ölkonzentration, vorzugsweise die Fischölkonzentration, im Bereich von etwa 3 bis 30 Gew.-% der Emulsion, beträgt vorzugsweise 10 bis 20 Gew.-%, beispielsweise 10 Gew.-% und enthält Omega-3-Fettsäuren in einer Menge von mindestens 20 Gew.-%, bezogen auf die gesamten Fettsäuren des Öles. Glycerin ist in einer Menge von etwa 1 bis 5 Gew.-%, bezogen auf die Emulsion, vorzugsweise von 2,5 Gew.-% enthalten. Der Emulgator, vorzugsweise Eilecithin, liegt in einer Menge von etwa 0,1 bis 6 Gew.-%, vorzugsweise 0,4 bis 2,0 Gew.-%, bezogen auf die Emulsion, vor.

Erfindungsgemäß wird die Emulsion dem Patienten intravenös verabreicht, wobei die intravenöse Verabreichung über einen zentralvenösen Zugang oder über eine periphere Vene erfolgen kann. Die erfindungsgemäß verwendete Emulsion ist zur Behandlung von Colitis ulcerosa wertvoll und wird zur Behandlung von Colitis ulcerosa eingesetzt.

Ein Vorteil der erfindungsgemäßen Verwendung besteht darin, daß eine Emulsion, die eine oder mehrere mehrfach ungesättigte, langkettige Omega-3-Fettsäuren, deren pharmazeutisch verträgliche Ester oder Salze enthält, oder die im wesentlichen aus Ölen, vorzugsweise Fischölen besteht, die mindestens 20 Gew.-% Omega-3-Fettsäuren enthalten, für die Patienten, die nicht auf Omega-6-Fettsäuren enthaltende Fettemulsionen reagieren, therapeutische Vorteile liefert. Ferner wird durch die parenterale Verabreichung der Fettemulsion schnell eine pharmazeutisch wirksame Menge an Omega-3-Fettsäuren zur Behandlung von entzündeten Stellen im Patienten bereitgestellt, verglichen mit der enteralen Verabreichung, wo das Verdauungssystem die Zeit erhöht und die Wahrscheinlichkeit herabsetzt, daß Omega-3-Fettsäuren die entzündeten Bereiche im Patienten erreichen.

Die durch die erfindungsgemäß verwendete Emulsion am Patienten erzielten Effekte können durch Beobachten und Vergleichen der Blutfettprofile und durch Blut- und Harn-Prostaglandin-Messungen vor und nach intravenöser Therapie bestimmt werden. Zusätzliche Messungen, einschließlich anthropometrische Messungen, d.h. Gewicht/Höhe, mittlerer Armmuskelumfang/Hautfaltendicke, metabolische Messungen und Ermittlung von VO₂ und VCO₂, respiratorischem Quotienten (RQ), Ruheenergieaufwand (REE) und cardiorespiratorische Reaktion auf Belastung können ebenfalls durchgeführt werden. Bei Patienten mit respiratorischer Funktionsstörung kann auch die respiratorische Muskelkraft gemessen werden und weiterhin können pulmonale Funktionsuntersuchungen/Gasaustauschtests durchgeführt werden.

Die nachfolgenden Beispiele dienen der weiteren Erläuterung der Erfindung.

Zur Erzielung von für die erfindungsgemäß verwendete Emulsion geeigneten hochraffinierten Fischöle, die mit Omega-3-Fettsäuren angereichert sind, wurden Fischöle von Kaltwasser-Meeresfischen, beispielsweise gemäß den Verfahren, wie sie in der DE-PS 37 22 540, insbesondere Beispiele 1 bis 4 beschrieben sind, behandelt.

### Beispiel 1

Unter Verwendung des gemäß Beispiel 5 der DE-PS 37 22 540 beschriebenen Verfahrens wurde eine Fettemulsion der in Tabelle I ersichtlichen Zusammensetzung hergestellt.

Die Mengen der Bestandteile sind in Milligramm der Komponente pro Milliliter Emulsion angegeben.

**Tabelle I**

| KOMPONENTE | mg/ml |
|---|---|
| Fischöle | 100 |
| Glycerin | 25 |
| Eilecthin | 12 |
| Wasser für Injektionszwecke | als Rest |

Die Fischöl-Fettemulsion hatte die aus Tabelle II ersichtliche Fettsäure-Zusammensetzung.

**Tabelle II**

| Fettsäure | mg/ml |
|---|---|
| C_{14:0} | 4,80 |
| C_{16:0} | 13,13 |
| C_{16:1} | 7,46 |
| C_{18:0} | 2,50 |
| C_{18:1} | 11,20 |
| C_{18:2} | 2,30 |
| C_{18:3} | 0,71 |
| C_{20:1} | 1,09 |
| C_{20:4} | 1,70 |
| C_{20:5} | 16,19 |
| C_{22:1} | 0,17 |
| C_{22:4} | 0,54 |
| C_{22:5} | 2,28 |
| C_{22:6} | 24,46 |

### Beispiel 2

Beispiel 1 wurde wiederholt mit der Ausnahme, daß anstelle einer 10 %igen Emulsion eine 20 %ige Emulsion hergestellt wurde.

### Beispiel 3

Beispiel 1 wurde wiederholt mit der Ausnahme, daß anstelle einer 10 %igen Emulsion eine 30 %ige Emulsion hergestellt wurde.

### Beispiel 4

Eine erfindungsgemäß zu verwendende Emulsion hatte die folgende Zusammensetzung, wobei die Mengen der Bestandteile in g des Bestandteiles pro 1 Emulsion angegeben sind:

| | |
|---|---|
| Fischöle | 100 g/l |
| Glycerin | 25 g/l |
| Eilecithin | 12 g/l |
| Wasser für Injektionszwecke | als Rest |

Die Fischöl-Emulsion hatte die aus Tabelle III ersichtliche Fettsäure-Zusammensetzung:

**Tabelle III**

| Säuren | g/l |
|---|---|
| Myristinsäure | 4,55 |
| Palmitinsäure | 9,03 |
| Palmitoleinsäure | 7,62 |
| Stearinsäure | 1,79 |
| Ölsäure | 11,50 |
| Linolsäure | 2,80 |
| Linolensäure | 1,65 |
| Arachidonsäure | 1,50 |
| C_{20:1} | 0,90 |
| C_{20:5} | 21,10 |
| C_{22:1} | 0,22 |
| C_{22:4} | 0,14 |
| C_{22:5} | 2,83 |
| C_{22:6} | 21,48 |
| Sonstige | 14,17 |

### Beispiel 5

Eine 36 Jahre alte Patientin, die unter einer Colitis ulcerosa und starken Steroidnebenwirkungen litt, erhielt im Abstand von 2 Monaten die im Beispiel 4 hergestellte Fischölemulsion.

Unter den Bedingungen einer mäßigen Krankheitsaktivität erhielt die Patientin über einen Zeitraum von 9 Tagen 200 ml der Fischölemulsion pro Tag (2 x 100 ml über je 3 Stunden) infundiert. Parallel dazu wurde die Dosis der verabreichten Steroide deutlich reduziert. Während der neuntägigen Fischölinfusion kam es zu einem vollständigen Rückgang der Krankheitsaktivität, gemessen an Stuhlfrequenz, Tenesmus und Rektalblutungen.

Zwei Monate nach Beendigung der ersten Infusionsperiode erlitt die Patientin ein Rezidiv mit intestinalen und extraintestinalen Manifestationen und die Infusion der Fischölemulsion wurde wieder aufgenommen. Die Infusionsperiode betrug 29 Tage, wobei eine Maximaldosierung von 300 ml pro Tag verwendet wurde. Auch während dieser zweiten Infusionsperiode wurde eine deutliche Verbesserung der entzündlichen Darmerkrankung festgestellt. Die pathologischen Amylase- und Lipasewerte gingen innerhalb von 3 bzw. 14 Tagen auf Normalwerte zurück. Systemische Entzündungszeichen wie das C-reaktive Protein verbesserten sich innerhalb von 3 Wochen auf Normalwerte. Die diffusen Abdominalschmerzen verschwanden langsam innerhalb von 2 Wochen und Blutstuhlkontamination sowie Tenesmus nahmen in ihrer Häufigkeit deutlich ab.

### Beispiel 6

Beispiel 4 wurde wiederholt mit der Ausnahme, daß anstelle einer 10 %igen Fettemulsion eine 15 %ige Fettemulsion hergestellt wurde. Bei Verwendung dieser Emulsion zur Behandlung von Colitis ulcerosa wurden Ergebnisse erhalten, die mit den in Beispiel 5 beschriebenen Ergebnissen vergleichbar waren.

### Beispiel 7

Beispiel 4 wurde wiederholt mit der Ausnahme, daß anstelle einer 10 %igen Fettemulsion eine 5 %ige Fettemulsion hergestellt wurde.

### Beispiel 8

Beispiel 4 wurde wiederholt mit der Ausnahme, daß anstelle einer 10 %igen Fettemulsion eine 20 %ige Fettemulsion hergestellt wurde. Bei Verwendung dieser Emulsion zur Behandlung von Colitis ulcerosa wurden Ergebnisse erhalten, die mit den in Beispiel 5 beschriebenen Ergebnissen vergleichbar waren.

### Beispiel 9

Beispiel 1 wurde wiederholt mit der Ausnahme, daß anstelle einer 10 %igen Emulsion eine 20 %ige Emulsion hergestellt wurde. Bei Verwendung dieser Emulsion zur Behandlung von Colitis ulcerosa wurden Ergebnisse erhalten, die mit den in Beispiel 5 beschriebenen Ergebnissen vergleichbar waren.

## Patentansprüche

1. Verwendung einer Emulsion, enthaltend eine oder mehrere mehrfach ungesättigte, langkettige Omega-3-Fettsäuren oder deren pharmazeutisch verträgliche Ester oder Salze sowie übliche Hilfs- und Zusatzstoffe zur Herstellung eines parenteral zu verabreichenden Arzneimittels zur Behandlung von Colitis ulcerosa.

2. Verwendung nach Anspruch 1, dadurch gekennzeichnet, daß die Omega-3-Fettsäuren 18 bis 22 Kohlenstoffatome aufweisen.

3. Verwendung nach Anspruch 1 oder 2, dadurch gekennzeichnet, daß die Omega-3-Fettsäure, Eicosapentaensäure und/oder Docosahexaensäure, vorzugsweise Eicosapentaensäure ist.

4. Verwendung nach einem oder mehreren der Ansprüche 1 bis 3, dadurch gekennzeichnet, daß die Ester als Ethylester oder Glycerinester, vorzugsweise als Triglyceride vorliegen.

5. Verwendung nach einem oder mehreren der Ansprüche 1 bis 4, dadurch gekennzeichnet, daß in den Emulsionen die Omega-3-Fettsäuren oder deren Ester oder Salze in Mengen von 1 bis 40 Gew.-% vorliegen.

6. Verwendung nach Anspruch 5, dadurch gekennzeichnet, daß die Omega-3-Fettsäuren oder deren Ester oder Salze in Mengen von 3 bis 30 Gew.-% vorliegen.

7. Verwendung nach einem oder mehreren der Ansprüche 1 bis 6, dadurch gekennzeichnet, daß die Omega-3-Fettsäuren in Form von Ölen, vorzugsweise Fischöl vorliegen.

8. Verwendung nach Anspruch 5, dadurch gekennzeichnet, daß die Emulsion aus 3 bis 30 Gew.-% Fischölen, die von 20 bis 100 Gew.-% Omega-3-Fettsäuren, bezogen auf die Gesamtfettsäuren, enthalten, 1 bis 5 Gew.-% Glycerin, 0,1 bis 6 Gew.-% mindestens eines Emulgators und Wasser für Injektionszwecke besteht.

9. Verwendung nach Anspruch 8, dadurch gekennzeichnet, daß die Fischöle von 40 bis 100 Gew.-% Omega-3-Fettsäuren, bezogen auf die Gesamtfettsäuren, enthalten.

10. Verwendung nach Anspruch 8 oder 9, dadurch gekennzeichnet, daß die Fischöle 10 bis 20 Gew.-% der Emulsion ausmachen.

11. Verwendung nach einem oder mehreren der Ansprüche 1 bis 10, dadurch gekennzeichnet, daß als Emulgator Phospholipide pflanzlichen oder tierischen Ursprungs verwendet werden.

12. Verwendung nach Anspruch 11, dadurch gekennzeichnet, daß als Emulgator Eilecithin, Eiphosphatide, Sojaphosphatide, Sojalecithin und andere Phospholipide verwendet werden.

13. Verwendung nach Anspruch 12, dadurch gekennzeichnet, daß als Emulgator Eilecithin verwendet wird.

14. Verwendung nach einem oder mehreren der Ansprüche 1 bis 13, dadurch gekennzeichnet, daß der Emulgator in einer Menge von 0,4 bis 2 Gew.-%, bezogen auf die Emulsion, verwendet wird.

15. Verwendung nach einem oder mehreren der Ansprüche 1 bis 13, dadurch gekennzeichnet, daß die Tagesdosis 1 bis 30 g Omega-3-Fettsäuren beträgt.

## Claims

1. Use of an emulsion containing a plurality of multiply unsaturated, long-chain omega-3 fatty acids or their pharmaceutically well-tolerated esters or salts, as well as the usual auxiliary substances and additives, for the manufacture of a parenterally administered medicinal substance for the treatment of ulcerative colitis.

2. Use according to claim 1, characterised in that the omega-3 fatty acids have 18 to 22 carbon atoms.

3. Use according to claim 1 or 2, characterised in that omega-3 fatty acid is eicosapentaenoic acid and/or docosahexaenoic acid, but preferably eicosapentaenoic acid.

4. Use according to one or more of claims 1 to 3, characterised in that the esters are present as ethyl ester or glycerol ester, but preferably as triglycerides.

5. Use according to one or more of claims 1 to 4, characterised in that the omega-3 fatty acids or their esters or salts are present in the emulsions in quantities of 3 to 30%/w.

6. Use according to claim 5, characterised in that the omega-3 fatty acids or their esters or salts are present in the emulsions in quantities of 3 to 30%/w.

7. Use according to one or more of claims 1 to 6, characterised in that the omega-3 fatty acids are present in the form of oils, preferably fish oils.

8. Use according to claim 5, characterised in that the emulsion consists of 3 to 30%/w fish oils which contain 20 to 100%/w omega-3 fatty acids with reference to the total fatty acids, 1 to 5%/w glycerol, 0.1 to 6%/w of at least one emulsifier, and water for injection purposes.

9. Use according to claim 8, characterised in that the fish oils contain from 40 to 100%/w omega-3 fatty acids with reference to the total fatty acids.

10. Use according to claim 8 or 9, characterised in that the fish oils constitute about 10 to 20%/w of the emulsion.

11. Use according to one or more of claims 1 to 10, characterised in that phospholipids of vegetable or animal origin are used as the emulsifier.

12. Use according to claim 11, characterised in that egg lecithin, egg phosphatides, soya phosphatides, soya lecithin and other phosphatides are used as the emulsifier.

13. Use according to claim 12, characterised in that egg lecithin is used as the emulsifier.

14. Use according to one or more of claims 1 to 13, characterised in that the emulsifier is used in a quantity of 0.4 to 2%/w with reference to the emulsion.

15. Use according to one or more of claims 1 to 13, characterised in that the daily dose is 1 to 30 g omega-3 fatty acids.

## Revendications

1. Utilisation d'une émulsion contenant un ou plusieurs acides gras oméga-3 à longues chaînes plusieurs fois insaturés ou encore leurs esters ou leurs sels pharmaceutiquement acceptables ainsi que des adjuvants et des additifs habituels pour la préparation d'un médicament à administrer par voie parentérale pour le traitement de Colitis ulcerosa.

2. Utilisation selon la revendication 1, caractérisée en ce que les acides gras oméga-3 à longues chaînes présentent de 18 à 22 atomes de carbone.

3. Utilisation selon la revendication 1 ou 2, caractérisée en ce que l'acide gras oméga-3 est l'acide éicosapentaénoïque et/ou docosahexaénoïque, de préférence l'acide éicosapentaénoïque.

4. Utilisation selon une ou plusieurs des revendications 1 à 3, caractérisée en ce que les esters sont présents sous forme d'esters éthyliques ou d'esters de glycérol, de préférence sous forme de triglycérides.

5. Utilisation selon une ou plusieurs des revendications 1 à 4, caractérisée en ce que, dans les émulsions, les acides gras oméga-3 ou encore leurs esters ou leurs sels sont présents dans des quantités de 1 à 40% en poids.

6. Utilisation selon la revendication 5, caractérisée en ce que les acides gras oméga-3 ou encore leurs esters ou leurs sels sont présents dans des quantités de 3 à 30% en poids.

7. Utilisation selon une ou plusieurs des revendications 1 à 6, caractérisée en ce que les acides gras oméga-3 sont présents sous forme d'huiles, de préférence d'huiles de poissons.

8. Utilisation selon la revendication 5, caractérisée en ce que l'émulsion est constituée par des huiles de poissons, à concurrence de 3 à 30% en poids, qui contiennent de 20 à 100% en poids d'acides gras oméga-3, rapportés à la quantité totale d'acides gras, par du glycérol à concurrence de 1 à 7% en poids, par au moins un émulsifiant à concurrence de 0,1 à 6% en poids et par de l'eau à des fins d'injection.

9. Utilisation selon la revendication 8, caractérisée en ce que les huiles de poissons contiennent des acides gras oméga-3 à concurrence de 40 à 100% en poids, rapportés à la quantité totale d'acides gras.

10. Utilisation selon la revendication 8 ou 9, caractérisée en ce que les huiles de poissons représentent de 10 à 20% en poids de l'émulsion.

11. Utilisation selon un ou plusieurs des revendications 1 à 10, caractérisée en ce qu'on utilise, comme émulsifiants, des phospholipides d'origine végétale ou animale.

12. Utilisation selon la revendication 11, caractérisée en ce qu'on utilise comme émulsifiant, de la lécithine d'oeuf, des phosphatides d'oeuf, des phosphatides de soja, de la lécithine de soja et d'autres phospholipides.

13. Utilisation selon la revendication 12, caractérisée en ce qu'on utilise comme émulsifiant, de la lécithine d'oeuf.

14. Utilisation selon une ou plusieurs des revendications 1 à 13, caractérisée en ce qu'on utilise l'émulsion en une quantité de 0,4 à 2% en poids rapportés à l'émulsion.

15. Utilisation selon un ou plusieurs des revendications 1 à 13, caractérisée en ce que la dose quotidienne s'élève de 1 à 30 g d'acides gras oméga-3.
